# EUROPÄISCHE PATENTSCHRIFT

(11) **EP 2 861 197 B1**
(45) Veröffentlichungstag und Bekanntmachung des Hinweises auf die Patenterteilung: **27.12.2017**
(21) Anmeldenummer: 13737159.7
(22) Anmeldetag: 14.06.2013
(51) Int. Cl.: A61G 13/10, A61G 13/12, A61B 17/88, A61G 13/00

(54) **EINRICHTUNG ZUM FIXIEREN EINES FEMURS IN DER HÜFTENDOPROTHETIK**
DEVICE FOR FEMUR SUPPORT DURING HIP ENDOPROSTHETIC
DISPOSITIF DE FIXATION D'UN FÉMUR DANS LE DOMAINE DE LA POSE D'ENDOPROTHÈSES DE LA HANCHE

(30) Priorität: 18.06.2012 DE 102012105264
(43) Veröffentlichungstag der Anmeldung: 22.04.2015
(73) Patentinhaber: Maquet GmbH, 76437 Rastatt (DE)
(72) Erfinder: KRICKEBERG, Thomas, 76307 Karlsbad (DE); BRAUER, Marcel, 77815 Bühl (DE); DÖRR, Holger, 76437 Rastatt (DE)
(74) Vertreter: Zacco GmbH
(86) Internationale Anmeldenummer: PCT/EP2013/062397
(87) Internationale Veröffentlichungsnummer: WO 2013/189854

(56) Entgegenhaltungen:
- WO-A2-2006/028788
- WO-A2-2007/021806
- US-A1- 2002 157 186

## Beschreibung

Die Erfindung betrifft eine Einrichtung zum Fixieren eines Femurs in der Hüftendoprothetik nach dem Oberbegriff des Anspruchs 1.

Während einer Operation zur minimalinvasiven Hüftendoprothetik mit anteriorem Zugang wird der Femurkopf abgetrennt und entfernt. Nach Abtrennen des Femurkopfes muss der verbleibende Femur stabilisiert und fixiert werden, um ein Absinken des Oberschenkels infolge des Eigengewichts des Beins zu verhindern. Ferner muss dafür gesorgt werden, dass der operierende Chirurg ständig optimalen Zugang zum Femur hat. Hierzu muss der Femur geringfügig angehoben werden.

Die vorstehend angeführten Maßnahmen werden im Stand der Technik in folgender Weise realisiert. Zunächst kommt eine Unterstützung des Femurs ohne eigens hierfür vorgesehene Hilfsmittel in Betracht. In diesem Fall hebt der Chirurg oder sein Assistent bei Bedarf den Femur manuell an.

Als technisches Hilfsmittel kommt häufig eine Polsterrolle zur Anwendung, die unterhalb des Oberschenkels angeordnet wird, um den Femur indirekt zu unterstützen. Auch eine indirekte Unterstützung mittels einer geeigneten, unterhalb des Gesäßes angeordneten Stütze ist denkbar.

Aus dem Stand der Technik sind ferner Einrichtungen bekannt, die eine direkte Unterstützung am Knochen mittels einer eigens hierfür vorgesehenen Femurstütze ermöglichen. Eine solche Einrichtung ist beispielsweise in US 7 824 353 B2 bzw. WO 2006/028788 A2 beschrieben. Diese vorbekannte Einrichtung umfasst eine Femurstütze mit einem gebogenen Hakenteil und einen mit dem Hakenteil verbundenen Schaft. Der Schaft ist an einem länglichen Ausleger angebracht, der wiederum an einer vertikalen Säule um deren Längsachse verschwenkbar montiert ist. Die Säule ist mit einer Verstellvorrichtung gekoppelt, die es ermöglicht, die Säule und damit den Ausleger und die an dem Ausleger aufgenommene Femurstütze in vertikaler Richtung zu bewegen. Diese Bewegung erfolgt in einer Verstellebene, die senkrecht zur Liegefläche des Operationstisches und parallel zu dessen Längsrichtung liegt. Zur Realisierung dieser Bewegung weist die Verstellvorrichtung einen in der Verstellebene liegenden Drehschaft auf, der beispielsweise mittels einer Kurbel betätigt wird, um so die Femurstütze geradlinig in vertikaler Richtung zu bewegen.

Die in der US 7 824 353 B2 offenbarte Lösung, die Femurstütze in der Verstellebene zu bewegen, ist vergleichsweise aufwändig. So bereitet es insbesondere Mühe, den in der Verstellebene liegenden Drehschaft beispielsweise mittels einer Kurbel zu betätigen, um die Femurstütze in der Verstellebene zu bewegen. Auch die geradlinige Bewegung der Femurstütze in der Verstellebene ist nicht immer geeignet, den Femur in der gewünschten Weise zu stabilisieren.

Die WO 2007/021806 A2 offenbart eine weitere Femurstützenlösung, bei der die Handkurbel durch einen Kraftantrieb ersetzt wird. Jedoch wird auch hier die Femurstütze geradlinig bewegt. Die US 2002/0157186 A1 beschreibt eine Einrichtung zum Abstützen der Hüfte eines Patienten.

Aufgabe der Erfindung ist es, eine zum Fixieren eines Femurs bestimmte Einrichtung eingangs erläuterter Art so weiterzubilden, dass sie insbesondere im Hinblick auf die Verstellung der Femurstütze einfach zu handhaben ist.

Die Erfindung löst diese Aufgabe durch die Einrichtung mit den Merkmalen des Anspruchs 1. Vorteilhafte Weiterbildungen sind in den Unteransprüchen gegeben.

Die erfindungsgemäße Einrichtung zeichnet sich dadurch aus, dass die Verstellvorrichtung eine senkrecht zu der Verstellebene liegende Drehachse aufweist, um welche die Halterung, an der die Säule des Hebelarms angebracht ist, mittels des Antriebs drehbar ist, um die Säule in der Verstellebene zu verschwenken. Mit Verstellebene ist dabei eine vertikale Ebene gemeint, die senkrecht zur Liegefläche des Operationstisches und parallel zu dessen Längsrichtung liegt. Dementsprechend verläuft die vorstehend genannte Drehachse horizontal und quer zur Liegefläche des Operationstisches.

Da die Erfindung eine Schwenkbewegung der Femurstütze vorsieht, lässt sich Ietztere besonders einfach anheben und absenken, beispielsweise indem die bei einem Operationstisch ohnehin vorgesehene Beinplattenfunktion zum Schwenken der Femurstütze genutzt wird. So verfügt ein Operationstisch häufig über eine Verstellvorrichtung, die ausgebildet ist, eine Beinplatte in vertikaler Richtung zu verschwenken. Eine solche Verstellvorrichtung hat beispielsweise eine für die Aufnahme einer Beinplatte geeignete Schnittstelle und/oder eine Profilschiene sowie einen Antriebsmotor, der die Profilschiene um eine Drehachse verschwenkt. Da bei der minimalinvasiven Hüftendoprothetik keine Beinplatten eingesetzt werden, kann eine solche motorische Verstellvorrichtung erfindungsgemäß nun dazu genutzt werden, die Femurstütze z.B. an der Profilschiene zu montieren und so deren Schwenkbewegung in der Verstellebene zu ermöglichen. Dies erleichtert dem Operateur die Handhabung der Einrichtung erheblich. Auch ist ein erheblicher wirtschaftlicher Nutzen gegeben, da die ohnehin vorhandene motorische Verstellvorrichtung verwendet werden kann, die Femurstütze in der gewünschten Weise zu bewegen.

Vorzugsweise hat der Hakenteil der Femurstütze einen senkrecht von dem Schaft abstehenden, geraden Abschnitt und einen an den geraden Abschnitt anschließenden, im Wesentlichen S-förmigen Abschnitt mit einem Endstück, das die Auflage für den zu fixierenden Femur bildet. Die Spitze dieses Endstück ist vorzugsweise abgerundet, um eine Schädigung des umliegenden Gewebes zu vermeiden.

In einer bevorzugten Ausgestaltung hat das Endstück des S-förmigen Abschnitts auf seiner Unterseite mindestens eine Einkerbung, in der ein Operationsinstrument abstützbar ist. Vorzugsweise sind mehrere solcher Einkerbungen vorgesehen, in denen beispielsweise so genannte Hohmann-Haken oder vergleichbare Wundspreitzer abgestützt werden können. Durch die Abstützung dieser Instrumente an dem Endstück wird die Gefahr von Weichteilverletzungen und Schädigungen am Knochen minimiert.

Vorzugsweise liegen der Schaft, der gerade Abschnitt des Hakenteils und ein an den geraden Abschnitt anschließender Teil des S-förmigen Abschnitts in einer Ebene, während das Endstück des S-förmigen Abschnitts in eine von dieser Ebene weg weisende Richtung gekrümmt ist. Diese Form der Femurstütze ermöglicht eine besonders stabile Anlage am Knochen zwischen Trochanter major und Trochanter minor. Es versteht sich dabei von selbst, dass zur Behandlung des rechten und des linken Femurs zwei Varianten der Femurstütze mit spiegelverkehrter Formgebung benötigt werden.

Vorteilhaft hat das gekrümmte Endstück des S-förmigen Abschnitts auf seiner in die genannte Richtung weisenden Seite eine Ausnehmung. Diese Ausnehmung befindet sich also am inneren Radius des Endstücks und hat den Zweck, Kontakt mit Weichteilen im Bereich der Auflagefläche des Endstücks zu vermeiden.

Vorzugsweise ist der Hakenteil der Femurstütze aus einem Profil gebildet, das einen flachen Profilquerschnitt hat. Dieser flache Profilquerschnitt führt dazu, dass der Hakenteil federnd ausgebildet ist, wodurch erhöhte Spannungen auf den Knochen abgefedert werden.

In einer besonders bevorzugten Ausführung ist der flache Profilquerschnitt im Bereich des Endstücks gegenüber dem flachen Profilquerschnitt im Bereich des restlichen Hakenteils um einen Winkel verdreht, der zwischen 10° und 30° liegt. Dies bedeutet, dass das Endstück um den vorstehend genannten Winkel gegenüber der Horizontalen verdreht ist. Diese verdrehte bzw. abgeschrägte Formgebung lässt Winkelabweichungen des Oberschenkelknochens infolge der Absenkung des Beins zu. Sie ermöglicht ferner den Femur vorzugsweise unter Anwendung der Beinplattenfunktion des Operationstisches in Adduktion und Abduktion zu verstellen. Außerdem sorgt diese Formgebung dafür, dass der Oberschenkelknochen stets eine optimale Auflagefläche am Endstück der Femurstütze hat.

Während der Hakenteil federnd ausgebildet ist, ist der Schaft der Femurstütze vorzugsweise biegesteif. Sowohl der Hakenteil als auch der Schaft der Femurstütze sind beispielsweise aus Edelstahl gefertigt.

Vorzugsweise weist die Halterung mindestens eine Buchse zur formschlüssigen Aufnahme der Säule des Hebelarms auf. Die Säule kann somit einfach in die Buchse gesteckt werden, um den Hebelarm an der Halterung zu befestigen. Sind mehrere Buchsen vorgesehen, so kann der Hebelarm wahlweise in eine dieser Buchsen gesteckt werden, um ihn in der gewünschten Weise zu positionieren.

In einer besonders bevorzugten Ausführung haben die Buchse und die Säule einander entsprechende polygonale Querschnitte, die jeweils eine derartige Symmetrie aufweisen, dass die Säule in mindestens zwei verschiedenen Ausrichtungen in der Buchse aufnehmbar ist. In dieser Ausführung hat die Säule ferner zwei gerade, parallel zueinander versetzte Säulenabschnitte, die über ein abgewinkeltes Übergangsstück miteinander verbunden sind. Damit ist es beispielsweise möglich, denjenigen Säulenabschnitt, an dem der Ausleger des Hebelarms angebracht ist, näher an den auf dem Operationstisch liegenden Patienten heranzubringen oder ihn weiter von dem Patienten zu entfernen. Haben die Buchse und die Säule beispielsweise rechteckige Querschnitte, so kann die Säule in mindestens zwei verschiedenen Ausrichtungen, die in einem Winkel von 180° um die Säulenlängsachse gedreht sind, in die Buchse eingesteckt werden. In Abhängigkeit der gerade gewählten Ausrichtung hat dann der parallel versetzte Säulenabschnitt, an dem der Ausleger montiert ist, einen größeren oder kleinen Abstand von dem Patienten.

Vorzugsweise ist der Ausleger mit der Säule um deren Längsachse schwenkbar gekoppelt. Diese schwenkbare Ankoppelung des Auslegers kann beispielsweise dadurch erzielt werden, dass der Ausleger eine erste Kontaktfläche und die Säule eine der ersten Kontaktfläche zugewandte zweite Kontaktfläche aufweist und dass die beiden Kontaktflächen mittels eines entsprechenden Betätigungselementes, beispielsweise einer Griffschraube, in Anlage miteinander gebracht werden, um die Verschwenkbarkeit des Auslegers zu sperren. Als Kontaktflächen können beispielsweise Zahnflächen verwendet werden, die durch Zudrehen der Griffschraube aufeinander gepresst werden.

In einer bevorzugten Ausführungsform hat der Schaft einen balligen Endabschnitt, z.B. in Form eines entsprechend geformten Zapfens. Die Aufnahme des Auslegers ist in dieser Ausführungsform aus einer länglichen Nut gebildet ist, in die der ballige Endabschnitt des Schaftes formschlüssig einsteckbar ist. Die ballige Ausführung des Schaftendabschnittes erlaubt es, diesen mit einer gewissen Winkelabweichung in die Nut einführen zu können, um den Schaft der Femurstütze an dem Ausleger zu befestigen. Ein Vorteil dieser Ausgestaltung ist, dass der Endabschnitt des Schaftes kein Profil wie z.B. ein Vierkant aufweist, das passgenau zu seinem Gegenstück (hier der Nut) ausgerichtet werden muss, um überhaupt in das Gegenstück eingeführt werden zu können. Der Schaft des Femurhakens kann so sehr einfach unter Tolerierung von gewissen Winkelabweichungen an dem Ausleger befestigt werden. Dieser erleichtert Handhabung während der Operation erheblich.

In einer speziellen Ausführungsform ist der Ausleger vorzugsweise aus mindestens zwei Teilen gebildet, von denen ein Teil zur Längeneinstellung des Auslegers teleskopartig gegen den anderen Teil verschiebbar ist. Die Längeneinsteilbarkeit des Auslegers macht es noch einfacher, die Femurstütze wie gewünscht zu positionieren.

Um die Längeneinstellung zu arretieren, weist vorzugsweise einer der beiden Teile des Auslegers ein Rastelement und der andere Teil mehrere Rastöffnungen zur Aufnahme des Rastelementes auf.

Die Erfindung wird im Folgenden anhand der Figuren näher erläutert. Darin zeigen:
- Fig. 1: eine Seitenansicht einer an einem Operationstisch angebrachten Einrichtung, die ein Ausführungsbeispiel der Erfindung darstellt;
- Fig. 2: eine Unteransicht einer Femurstütze;
- Fig. 3: eine Seitenansicht der Femurstütze;
- Fig. 4: eine geschnittene Vorderansicht der Femurstütze;
- Fig. 5: eine Draufsicht der Femurstütze;
- Fig. 6: eine Seitenansicht eines aus einer Säule und einem Ausleger gebildeten Hebelarms;
- Fig. 7: eine Seitenansicht, welche die an dem Ausleger angebrachte Femurstütze zeigt;
- Fig. 8: eine weitere, teilweise geschnittene Ansicht, welche die in der Aufnahme des Auslegers angebrachte Femurstütze zeigt;
- Fig. 9: eine Draufsicht, die einen Teil der in der Aufnahme des Auslegers angebrachte Femurstütze zeigt;
- Fig. 10: eine perspektivische Ansicht einer Halterung, an welcher der Hebelarm anbringbar ist;
- Fig. 11: eine Schnittansicht der Halterung;
- Fig.12: eine teilweise geschnittene Seitenansicht einer abgewandelten Ausführungsform des Auslegers; und
- Fig. 13: eine Unteransicht des Auslegers nach Figur 12.

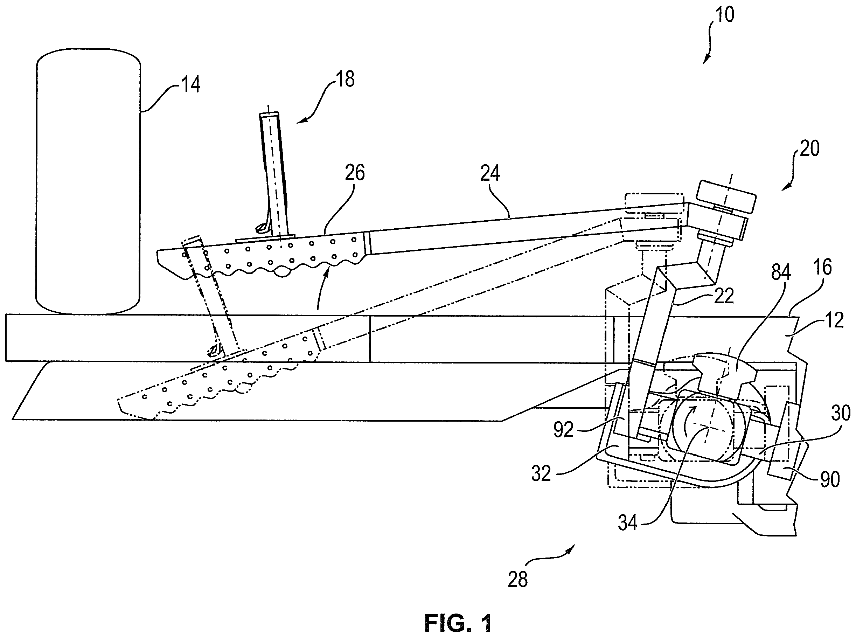

Figur 1 ist eine Seitenansicht, die eine erfindungsgemäße Einrichtung 10 als Ausführungsbeispiel zeigt. Die Einrichtung 10 dient dazu, während einer Operation zur minimalinvasiven Hüftendoprothetik einen nicht gezeigten Femur zu stützen. Sie ist an einem Operationstisch 12 montiert, auf dem der zu operierende Patient zu lagern ist. In Figur 1 ist ferner eine Stützrolle 14 gezeigt, die auf einer Lagerfläche 16 des Operationstisches 12 angebracht ist.

Die Einrichtung 10 umfasst eine später im Einzelnen beschriebene Femurstütze 18, einen Hebelarm 20, der aus einer Säule 22 und einem Ausleger 24 gebildet ist. Der Ausleger 24 hat eine Aufnahme 26, in der die Femurstütze 18 angebracht ist.

Die Einrichtung 10 umfasst ferner eine an einer Seitenfläche des Operationstisches 12 montierte Verstellvorrichtung 28, die dazu dient, den Hebelarm 20 in einer vertikalen Verstellebene zu verschwenken. In Figur 1 sind zwei Schwenkstellungen des Hebelarms 20 gezeigt, von denen eine mit gestrichelten Linien angedeutet ist. Die Verstellebene, in welcher der Hebelarm 20, wie in Figur 1 durch den Pfeil angedeutet, mittels der Verstellvorrichtung 28 verschwenkt wird, liegt senkrecht zur Liegefläche 16 des Operationstisches 12 und parallel zu dessen Längsrichtung. Die Verstellebene liegt damit parallel zur Zeichenebene der Figur 1.

Die Die Einrichtung 10 hat eine mit der Verstellvorrichtung 28 koppelbare Halterung 30, an welcher der Hebelarm 20 befestigt ist. Die Verstellvorrichtung 28 weist einen Antriebsmotor 32 auf, der dazu dient, die Halterung 30 um eine horizontale Drehachse 34 zu drehen, die senkrecht zu der vorstehend genannten Verstellebene liegt. Der Antriebsmotor 32 kann in dem vorliegenden Ausführungsbeispiel in einer anderen Anwendung auch dazu genutzt werden, eine nicht gezeigte Beinplatte in der Verstellebene zu verschwenken.

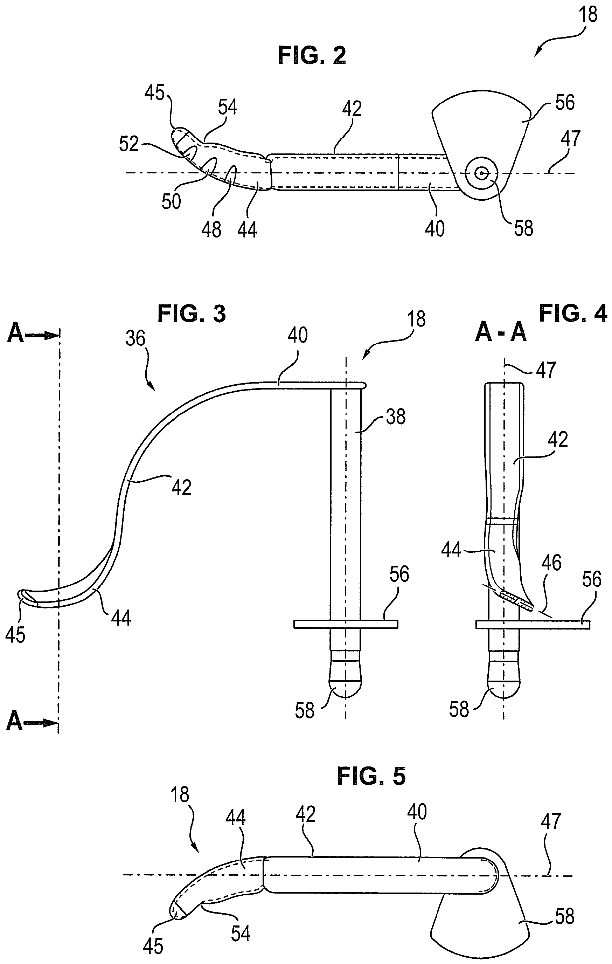

Im Folgenden wird unter Bezugnahme insbesondere auf die Figuren 2 bis 5 die Femurstütze 18 im Einzelnen beschrieben.

Die Femurstütze 18 ist, wie am besten in Figur 3 zu erkennen ist, aus einem Hakenteil 36 und einem Schaft 38 gebildet. Der Hakenteil 36 weist über seine gesamte Länge einen flachen Profilquerschnitt auf und ist damit federnd ausgebildet. Demgegenüber hat der Schaft 38 einen runden Querschnitt. Er ist somit biegesteif ausgebildet.

Der Hakenteil 36 hat einen senkrecht von dem Schaft 38 abstehenden, geraden Abschnitt 40, der in einen im Wesentlichen S-förmig gebogenen Abschnitt 42 übergeht. Am freien Ende des S-förmigen Abschnittes 42 befindet sich ein abgerundetes Endstück 44, das dazu bestimmt ist, am Oberschenkelknochen zwischen Trochanter major und Trochanter minor anzuliegen. Das Endstück 44 hat eine abgerundete Spitze 45 ohne scharfe Kanten. Es kann somit in die Wunde eingeführt werden, ohne das mit ihm in Kontakt kommende Gewebe zu schädigen.

Wie die Unteransicht nach Figur 2, die geschnittene Vorderansicht nach Figur 4 und die Draufsicht nach Figur 5 zeigen, ist die Femurstütze 18 im Wesentlichen so geformt, dass sie mit Ausnahme des Endstücks 44 im Wesentlichen in einer Ebene liegt. Diese Ebene ist in den Figuren 2, 4 und 5 senkrecht zur Zeichenebene angeordnet und dort mit dem Bezugszeichen 47 bezeichnet. Demgegenüber ist das Endstück 44 in eine Richtung gekrümmt, die von der Ebene 47 weg weist. Dies ist insbesondere in den Figuren 2 und 5 zu erkennen.

Zudem ist der flache Profilquerschnitt des Hakenteils 36 im Bereich des Endstücks 44 gegenüber dem flachen Profilquerschnitt im Bereich des restlichen Hakenteils um einen vorbestimmten Winkel verdreht. Diese Verdrehung ist in Figur 4 durch den mit der Linie 46 bezeichneten Querschnitt angedeutet, der unter dem vorbestimmten Winkel gegenüber der Horizontalen geneigt ist. In dem vorliegenden Ausführungsbeispiel beträgt der Neigungswinkel etwa 25°. Die Oberseite des Endstücks 44 bildet somit eine abgeschrägte Auflagefläche, die es erlaubt, das gestreckte Bein des Patienten in einem variablen Winkel (z.B. zwischen -10° und - 30°) gegenüber der Horizontalen abzusenken und dabei eine optimal unterstützende Auflage für den Femur zu bilden.

Der weitgehend vertikale Abschnitt des Hakenteils 36, der zwischen dem Endstück 44 und dem geraden Abschnitt 40 liegt, hat u.a. die Funktion, die Weichteile des Patienten vor Instrumentenkontakt zu schützen. Hierzu trägt der flache Profilquerschnitt des Hakenteils 36 bei.

Wie in Figur 2 gezeigt, hat das Endstück 44 des Hakenteils 36 auf seiner Unterseite mehrere Einkerbungen 48, 50 und 52. In diesen Einkerbungen 48, 50, 52 können Operationsinstrumente wie Hohmann-Haken oder Wundspreitzer abgestützt werden. Durch diese Abstützung werden Weichteilverletzungen und Knochenschädigungen vermieden.

Wie insbesondere den Figuren 2 und 5 zu entnehmen ist, weist das gekrümmte Endstück 44 des Hakenteils 36 auf der Seite, die in die Krümmungsrichtung weist (in Figur 2 links oben und in Figur 5 links unten), eine Aussparung 54 auf, die den Zweck hat, im Bereich der Auflagefläche Kontakt mit Weichteilen zu vermeiden.

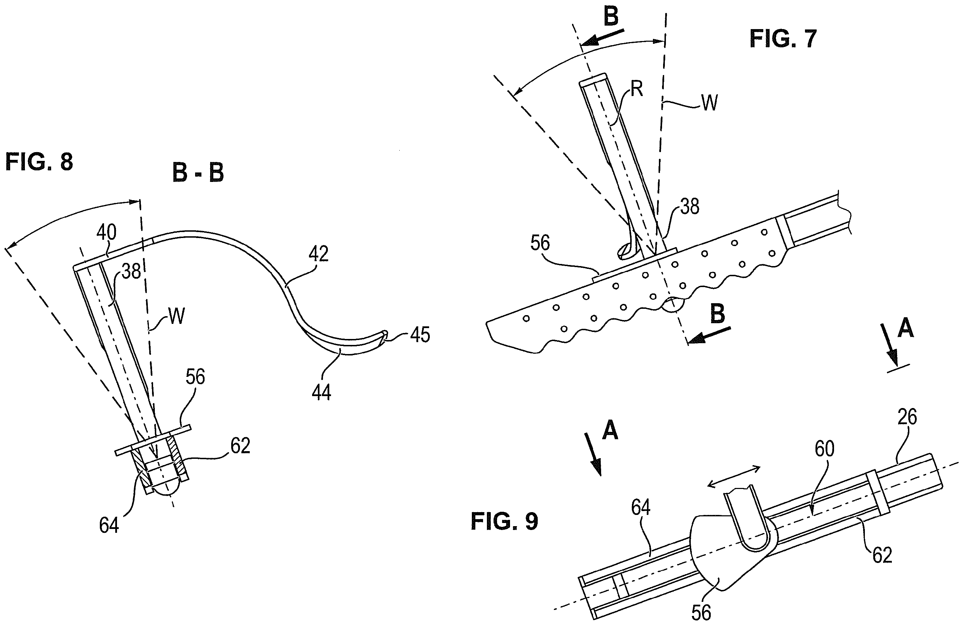

Wie am besten in den Figuren 3 und 4 zu erkennen ist, hat der Schaft 38 im Bereich seines freien Endes eine Auflageplatte 56, die dazu dient, sich auf der Aufnahme 26 des Hebelarms 20 abzustützen (vgl. Fig. 7). Ferner ist das freie Ende des Schafts 38 als balliges Endstück 58 ausgebildet, d.h. als ein Endstück, das konvex oder abgeschrägt zuläuft. Die Funktion dieses in Form eines balligen Zapfens ausgebildeten Endstücks 58 ist in den Figuren 7 und 8 veranschaulicht.

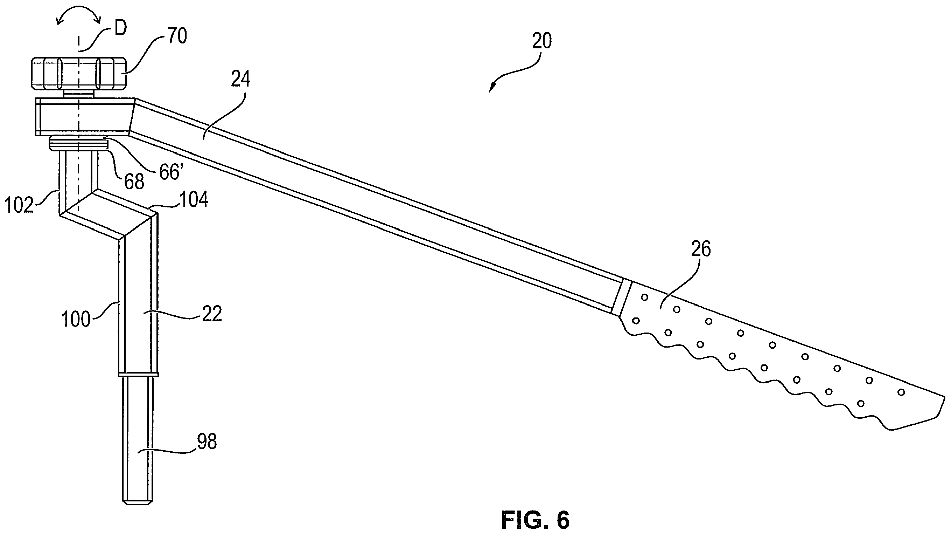

Die Figuren 7 und 8 zeigen die Femurstütze 18 in einem Zustand, in dem sie in die Aufnahme 26 des Hebelarms 20 eingesteckt ist. Der mit der Aufnahme 26 versehene Hebelarm 20 ist in Figur 6 nochmals in Alleinstellung dargestellt. Wie dort gezeigt, befindet sich die Aufnahme 26 am freien Ende des Auslegers 24 des Hebelarms 20.

Wie in Figur 9 gezeigt, ist die Aufnahme 26 aus einer länglichen Nut 60 gebildet, die zwei einander gegenüberliegende Anlageflächen 62 und 64 hat (vgl. auch Fig. 8). Die ballige Ausführung des Endstücks 58 ermöglicht es dem Operateur, den Schaft 38, der zugleich als Handgriff dient, mit einer tolerierbaren Winkelabweichung gegenüber einer vorbestimmten Einsteckrichtung in die Nut 60 einzuführen. Dieser Sachverhalt ist insbesondere in den Figuren 7 und 8 veranschaulicht, in denen die vorstehend genannte vorbestimmte Einsteckrichtung mit R und die dem gegenüber tolerierbare Winkelabweichung mit W bezeichnet ist. Im Unterschied beispielsweise zu einem Vierkant stellt das ballige Endstück 58 kein Profil dar, das passgenau an der Nut 60 ausgerichtet werden muss, um überhaupt in die Nut 60 eingeführt werden zu können. Vielmehr ermöglicht die ballige Ausführung des Endstücks 58, dass sich dieses an verschiedenen Stützpunkten auf den Anlageflächen 62 und 64 der Nut 60 abstützen kann, während es in die Nut 60 eingeführt wird. Dadurch wird der in den Figuren 7 und 8 dargestellte Winkelbereich W realisiert, innerhalb dessen der Schaft 38 beim Einstecken in die Nut 60 ausgerichtet werden kann, ohne so stark zu verkanten, dass ein Einführen des Endstücks 58 in die Nut 60 unmöglich wird.

Ist das Endstück 58 vollständig in der Nut 60 aufgenommen, so liegt es an den Anlageflächen 62 und 64 an. Außerdem liegt in diesem Zustand die Auflageplatte 56 auf der Oberseite der Nut 60 auf. Durch die Abstützkraft ist das Endstück 58 im eingesteckten Zustand sicher in der Nut 60 fixiert. Wie in Figur 9 durch den Doppelpfeil dargestellt, lässt sich so der Schaft 38 der Femurstütze 18 einfach und sicher an einer beliebigen Stelle längs der Nut 60 einstecken.

Wie in Figur 6 gezeigt, ist der Ausleger 24 um eine vertikale Drehachse D schwenkbar an der Säule 22 montiert. Die miteinander gekoppelten Teile des Auslegers 24 und der Säule 22 sind dabei jeweils mit einer Zahnfläche 66 bzw. 68 versehen, die sich über eine Griffschraube 70 aufeinander pressen lassen, um die Rotation des Auslegers 24 um die vertikale Schwenkachse D zu sperren. Der Ausleger 24 kann so in einer beliebigen Schwenkstellung arretiert werden.

Die in Figur 6 dargestellte Ausführungsform bietet die Möglichkeit, den Ausleger 24 bei Bedarf von dem Patienten weg zu schwenken, um einen besseren Zugang zum Femur zu erreichen.

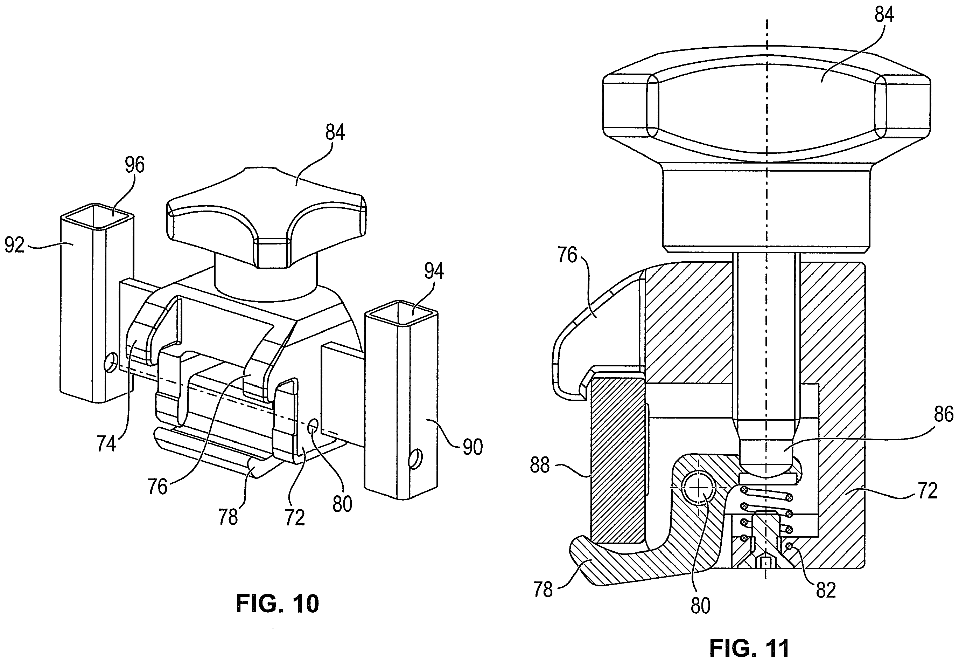

In den Figuren 10 und 11 ist die in der Einrichtung 10 vorgesehene Halterung 30 genauer dargestellt.

Die Halterung 30 hat ein Gehäuse 72, das im Querschnitt im Wesentlichen L-förmig ist. An dem Gehäuse 72 befinden sich zwei klauenförmige Vorsprünge 74 und 76, die mit einem Klemmstück 78 zusammenwirken. Das Klemmstück 78 ist auf einem Lagerbolzen 80 gelagert und über eine Druckfeder 82, die sich gegen das Gehäuse 72 und einen Schenkel des Klemmstücks 78 abstützt, in eine Offenstellung vorgespannt. In dieser Offenstellung haben die klauenförmigen Vorsprünge 74 und 76 einerseits und das Klemmstück 78 andererseits den größtmöglichen vertikalen Abstand voneinander.

In das Gehäuse 72 ist von oben eine Griffschraube 84 geschraubt, deren freies Ende 86 auf das Klemmstück 78 drückt. Wird die Griffschraube 84 zugedreht, so wird das Klemmstück 78 um den Lagerbolzen 80 in Figur 11 im Uhrzeigersinn verschwenkt.

Das Gehäuse 72 wird über die beiden klauenförmigen Vorsprünge 74, 76 und das Klemmstück 78 an einer Profilschiene 88 festgeklemmt, die an der Verstellvorrichtung 28 montiert ist. Hierzu wird das Gehäuse 72 in einem Zustand, in dem die Vorsprünge 74, 76 und das Klemmstück 78 den größtmöglichen vertikalen Abstand voneinander haben, so auf die Profilschiene 88 gesteckt, dass diese in dem Raum zwischen den Vorsprüngen 74, 76 und dem Klemmstück 78 aufgenommen wird. Dann wird die Griffschraube 84 zugedreht, um das Klemmstück 78 entgegen der von der Druckfeder 82 ausgeübten Vorspannkraft um den Lagerzapfen 80 zu verschwenken. Dadurch wird die Profilschiene 88 zwischen den Vorsprüngen 74, 76 und dem Klemmstück 78 festgeklemmt. Dabei liegt das Gehäuse 72 an vier Punkten an der Profilschiene 82 an, nämlich den beiden Vosprüngen 74, 76 und den unterhalb der Vorsprünge 74, 76 liegenden Gehäusestirnflächen.

Das Gehäuse 72 hat auf seinen einander abgewandten Seitenflächen zwei Buchsen 90 und 92. Die Buchsen 90, 92 haben jeweils eine im Querschnitt rechteckige Durchgangsöffnung 94 bzw. 96. Die Öffnungen 94 und 96 dienen der Aufnahme des in Figur 6 mit 98 bezeichneten freien Endes der Säule 22. Dieses freie Ende 98 der Säule 22 ist entsprechend der Querschnittsform der jeweiligen Öffnung 94, 96 als Vierkant ausgebildet. Es kann somit formschlüssig in jede der Öffnungen 94 und 96 eingesteckt werden. Zur Arretierung in derjeweiligen Öffnung 94, 96 weist das freie Ende 98 der Säule 22 beispielsweise einen Sicherungszapfen auf, der in den Figuren nicht dargestellt ist.

Aufgrund der Querschnittsform der Öffnungen 94, 96 und des Endstücks 98 kann letzteres in unterschiedlichen Ausrichtungen in die jeweilige Öffnung 94, 96 eingesteckt werden. Dies kann dazu genutzt werden, den Abstand, den die in Figur 6 gezeigte Schwenkachse D, um die der Ausleger 24 verschwenkbar ist, von dem Patienten hat, zu vergrößern oder zu verringern. Hierzu ist die Säule 22 aus zwei geraden, parallel zueinander versetzten Säulenabschnitten 100, 102 und einem abgewinkelten Übergangsstück 104 gebildet, das die zueinander versetzten Säulenabschnitte 100, 102 miteinander verbindet. Abhängig von der Ausrichtung des Endstücks 98 innerhalb der jeweiligen Buchse 94 bzw. 96 kann so der Säulenabschnitt 102, durch den die Schwenkachse D geht, näher an den Patienten herangebracht oder von diesem entfernt werden.

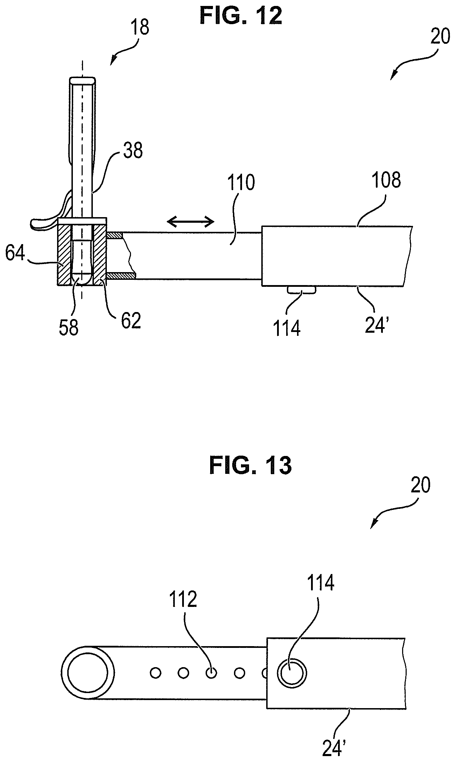

In den Figuren 12 und 13 ist eine abgewandelte Form des Auslegers des Hebelarms 20 dargestellt und dort mit 24' bezeichnet. Der Ausleger 24' ist aus einem ersten Teil 108 und einem zweiten Teil 110 gebildet, der teleskopartig verschiebbar in dem ersten Teil 108 aufgenommen ist, wie in Figur 12 durch den Doppelpfeil angedeutet ist. Indem der zweite Teil 110 aus dem ersten Teil 108 herausgezogen oder in diesen hineingeschoben wird, lässt sich die Länge des Auslegers 24' variieren.

Die Arretierung dieser Längeneinstellung erfolgt dadurch, dass ein an dem ersten Teil 108 ausgebildetes, in den Figuren 12 und 13 nicht gezeigtes Rastelement während des Verschiebens des zweiten Teils 110 selbsttätig in eine von mehreren Rastöffnungen 114 einrastet, die an dem zweiten Teil 110 ausgebildet sind. Über ein mit dem Rastelement zusammenwirkendes Bedienelement 114 lässt sich die Arretierung wieder lösen.

Die in den Figuren 12 und 13 dargestellte Ausführungsform zeichnet sich dadurch aus, dass sich das Lösen der Arretierung und das Verstellen der Länge des Auslegers 24' gleichzeitig mit einer Hand durchführen lässt.

## Patentansprüche

1. Einrichtung (10) zum Fixieren eines Femurs in der Hüftendoprothetik, umfassend
eine Femurstütze (18) mit einem gebogenen Hakenteil (36), der eine unterstützende Auflage für den zu fixierenden Femur im Bereich zwischen Trochanter major und Trochanter minor bildet, und einem mit dem Hakenteil (36) verbundenen Schaft (38),
einen Hebelarm (20) mit einer Säule (22) und einem Ausleger (24), der eine Aufnahme (26) zur Anbringung des Schafts (38) aufweist,
eine an einem Operationstisch (12) angebrachte Verstellvorrichtung (28), und eine mit der Verstellvorrichtung (28) koppelbare Halterung (30), an der die Säule (22) des Hebelarms (20) anbringbar ist,
wobei die Verstellvorrichtung (28) einen Antrieb (32) zum Bewegen der Säule (22) in einer Verstellebene hat, die senkrecht zur Liegefläche (16) des Operationstisches (12) und parallel zu dessen Längsrichtung liegt,
**dadurch gekennzeichnet, dass** die Verstellvorrichtung (28) eine senkrecht zu der Verstellebene liegende Drehachse (34) aufweist, um welche die Halterung (30) mittels des Antriebs (32) zum Bewegen der Säule (22) in der Verstellebene drehbar ist.

2. Einrichtung (10) nach Anspruch 1, **dadurch gekennzeichnet, dass** der Hakenteil (36) der Femurstütze (18) einen senkrecht von dem Schaft (38) abstehenden, geraden Abschnitt (40) und einen an den geraden Abschnitt (40) anschließenden, im Wesentlichen S-förmigen Abschnitt (42) mit einem Endstück (44) aufweist, das die Auflage für den zu fixierenden Femur bildet.

3. Einrichtung (10) nach Anspruch 2, **dadurch gekennzeichnet, dass** das Endstück (44) des S-förmigen Abschnitts (42) abgerundet ist.

4. Einrichtung (10) nach Anspruch 2 oder 3, **dadurch gekennzeichnet, dass** der Schaft (38), der gerade Abschnitt (40) des Hakenteils (36) und ein an den geraden Abschnitt (40) anschließender Teil des S-förmigen Abschnitts (42) in einer Ebene (A-A) liegen, und
das Endstück (44) des S-förmigen Abschnitts (42) in eine von dieser Ebene (A-A) weg weisende Richtung gekrümmt ist.

5. Einrichtung (10) nach einem der vorhergehenden Ansprüche, **dadurch gekennzeichnet, dass** der Hakenteil (36) der Femurstütze (18) aus einem Profil gebildet ist, das einen flachen Profilquerschnitt hat.

6. Einrichtung (10) nach Anspruch 5, **dadurch gekennzeichnet, dass** der flache Profilquerschnitt im Bereich des Endstücks (44) gegenüber dem flachen Profilquerschnitt im Bereich des restlichen Hakenteils (36) um einen Winkel verdreht ist, der zwischen 10° und 30° liegt.

7. Einrichtung (10) nach einem der vorhergehenden Ansprüche, **dadurch gekennzeichnet, dass** die Halterung (30) mindestens eine Buchse (94, 96) zur formschlüssigen Aufnahme der Säule (22) des Hebelarms (20) aufweist.

8. Einrichtung (10) nach Anspruch 7, **dadurch gekennzeichnet, dass** die Buchse (94, 96) und die Säule (22) einander entsprechende polygonale Querschnitte haben, die jeweils eine derartige Symmetrie aufweisen, dass die Säule (22) in mindestens zwei verschiedenen Ausrichtungen in der Buchse (94, 96) aufnehmbar ist, und
die Säule (22) zwei gerade, parallel zueinander versetzte Säulenabschnitte (100, 102) hat, die über ein abgewinkeltes Übergangsstück (104) miteinander verbunden sind.

9. Einrichtung (10) nach einem der vorhergehenden Ansprüche, **dadurch gekennzeichnet, dass** die Halterung (30) eine an dem Operationstisch (12) angebrachte Profilschiene (88) hat und dass die Halterung (30) mindestens einen klauenförmigen Vorsprung (74, 76), ein über ein Federelement (82) in eine Offenstellung vorgespanntes Klemmstück (78) und ein Betätigungselement (84) aufweist, mit dem das Klemmstück (78) unter Verringerung seines Abstands von dem Vorsprung (74, 76) entgegen der von dem Federelement (82) ausgeübten Vorspannkraft in eine Schließstellung bewegbar ist, wobei die Profilschiene (88) in der Offenstellung in dem Raum zwischen Vorsprung (74, 76) und Klemmstück (78) aufnehmbar und in der Schließstellung zwischen dem Vorsprung (74, 76) und dem Klemmstück (78) festgeklemmt ist.

10. Einrichtung (10) nach Anspruch 9, **dadurch gekennzeichnet, dass**
das Klemmstück (78) auf einem Lagerbolzen (80) zwischen seiner Offenstellung und seiner Schließstellung schwenkbar gelagert ist, und
das Betätigungselement eine von Hand betätigbare Griffschraube (84) ist, die zum Schwenken des Klemmstücks (80) in die Schließstellung entgegen der von dem Federelement (82) ausgeübten Vorspannkraft auf das Klemmstück (78) drückt.

11. Einrichtung (10) nach einem der vorhergehenden Ansprüche, **dadurch gekennzeichnet, dass** der Ausleger (24) mit der Säule (22) um deren Längsachse (D) schwenkbar gekoppelt ist.

12. Einrichtung (10) nach Anspruch 11, **dadurch gekennzeichnet, dass**
der Ausleger (24) eine erste Kontaktfläche (66) und die Säule (22) eine der ersten Kontaktfläche zugewandte zweite Kontaktfläche (68) aufweist, und
die beiden Kontaktflächen (66, 68) zum Sperren der Verschwenkbarkeit des Auslegers (24) in Anlage miteinander bringbar sind.

13. Einrichtung (10) nach einem der vorhergehenden Ansprüche, **dadurch gekennzeichnet, dass** der Schaft (38) einen balligen Endabschnitt (58) hat, und
die Aufnahme des Auslegers (24) aus einer länglichen Nut (60) gebildet ist, in die der ballige Endabschnitt (58) des Schafts (38) einsteckbar ist.

14. Einrichtung (10) nach einem der vorhergehenden Ansprüche, **dadurch gekennzeichnet, dass** der Ausleger (24') aus mindestens zwei Teilen (108, 110) gebildet ist, von denen ein Teil (110) zur Längeneinstellung des Auslegers (24') teleskopartig gegen den anderen Teil (108) verschiebbar ist.

15. Einrichtung (10) nach Anspruch 14, **dadurch gekennzeichnet, dass** einer der beiden Teile (108, 110) des Auslegers (24) ein Rastelement und der andere Teil mehrere Rastöffnungen (112) zur Aufnahme des Rastelementes (114) hat.

## Claims

1. A device (10) for fixing a femur in a hip endoprosthesis, comprising
a femur support (18) having a curved hook part (36) which in the area between the trochanter major and the trochanter minor forms an assisting support for the femur to be fixed, and having a shaft (38) that is connected to the hook part (36) ,
a lever arm (20) with a column (22) and an arm (24) which has a holder (26) for attaching the shaft (38),
an adjustment device (28) that is mounted on an operating table (12), and a mounting (30) which is coupleable to the adjustment device (28) and on which the column (22) of the lever arm (20) is mountable,
wherein the adjustment device (28) has a drive (32) for moving the column (22) in an adjustment plane situated perpendicularly with respect to the lying surface (16) of the operating table (12) and parallel to the longitudinal direction thereof,
**characterized in that** the adjustment device (28) has a rotational axis (34), situated perpendicularly with respect to the adjustment plane, about which the mounting (30) is rotatable by means of the drive (32) in order to move the column (22) in the adjustment plane.

2. The device (10) according to Claim 1, **characterized in that** the hook part (36) of the femur support (18) has a straight section (40) that protrudes perpendicularly from the shaft (38), and an essentially S-shaped section (42), adjoining the straight section (40), with an end piece (44) that forms the support for the femur to be fixed.

3. The device (10) according to Claim 2, **characterized in that**
the end piece (44) of the S-shaped section (42) is rounded.

4. The device (10) according to Claim 2 or 3, **characterized in that** the shaft (38), the straight section (40) of the hook part (36), and a portion of the S-shaped section (42) connected to the straight section (40) lie in a plane (A-A), and
the end piece (44) of the S-shaped section (42) is curved in a direction pointing away from this plane (A-A).

5. The device (10) according to one of the preceding claims,
**characterized in that** the hook part (36) of the femur support (18) is formed from a profile having a flat profile cross section.

6. The device (10) according to Claim 5, **characterized in that** the flat profile cross section in the area of the end piece (44) is rotated by an angle between 10° and 30° relative to the flat profile cross section in the area of the remaining hook part (36).

7. The device (10) according to one of the preceding claims,
**characterized in that** the mounting (30) has at least one socket (94, 96) for holding the column (22) of the lever arm (20) in a form-fit manner.

8. The device (10) according to Claim 7, **characterized in that** the socket (94, 96) and the column (22) have mutually corresponding polygonal cross sections, each having a symmetry such that the column (22) is accommodatable in at least two different orientations in the socket (94, 96), and
the column (22) has two straight column sections (100, 102) which are offset in parallel to one another, and which are connected to one another via an angled transition piece (104) .

9. The device (10) according to one of the preceding claims,
**characterized in that** the mounting (30) has a profile bar (88) that is mounted on the operating table (12), and the mounting (30) has at least one claw-shaped protrusion (74, 76), a clamping piece (78) that is pretensioned in an open position via a spring element (82), and an actuating element (84) via which the clamping piece (78) is movable into a closed position, against the pretensioning force exerted by the spring element (82), with reduction in the distance of the clamping piece from the protrusion (74, 76), wherein the profile bar (88) in the open position is accommodatable in the space between the protrusion (74, 76) and the clamping piece (78), and in the closed position is clamped between the protrusion (74, 76) and the clamping piece (78).

10. The device (10) according to Claim 9, **characterized in that** the clamping piece (78) is pivotably supported on a bearing bolt (80) between the open position and the closed position of the clamping piece, and the actuating element is a manually actuatable handle screw (84) which presses on the clamping piece (78), against the pretensioning force exerted by the spring element (82), in order to pivot the clamping piece (80) into the closed position.

11. The device (10) according to one of the preceding claims,
**characterized in that** the arm (24) is coupled to the column (22) so as to be pivotable about the longitudinal axis (D) of the column.

12. The device (10) according to Claim 11, **characterized in that** the arm (24) has a first contact surface (66), and the column (22) has a second contact surface (68) facing the first contact surface, and
the two contact surfaces (66, 68) may be brought into contact with one another in order to block the pivotability of the arm (24).

13. The device (10) according to one of the preceding claims,
**characterized in that** the shaft (38) has a convex end section (58), and the holder of the arm (24) is formed from an elongated groove (60) into which the convex end section (58) of the shaft (38) is insertable.

14. The device (10) according to one of the preceding claims,
**characterized in that** the arm (24') is formed from at least two parts (108, 110), one part (110) being displaceable against the other part (108) in a telescoping manner in order to adjust the length of the arm (24').

15. The device (10) according to Claim 14, **characterized in that** one of the two parts (108, 110) of the arm (24) has a detent element, and the other part has multiple detent openings (112) for accommodating the detent element (114).

## Revendications

1. Dispositif (10) pour fixer un fémur dans une endoprothèse de la hanche, comprenant
un support de fémur (18) avec une partie de crochet courbe (36) formant une base d'appui pour le fémur à fixer dans la zone entre le grand trochanter et le petit trochanter, et une tige (38) reliée à la partie de crochet (36),
un bras de levier (20) avec une colonne (22) et une console (24) présentant un logement (26) pour monter la tige (38),
un dispositif de réglage (28) monté sur une table d'opération (12) et une bride (30) pouvant être accouplée au dispositif de réglage (28) et sur laquelle la colonne (22) du bras de levier (20) peut être montée,
dans lequel le dispositif de réglage (28) présente un entraînement (32) pour déplacer la colonne (22) dans un plan de réglage qui est perpendiculaire à la surface de couchage (16) de la table d'opération (12) et parallèle à la direction longitudinale de celle-ci,
**caractérisé en ce que** le dispositif de réglage (28) présente un axe de rotation (34) perpendiculaire au plan de réglage et autour duquel la bride (30) peut être amenée à tourner au moyen de l'entraînement (32) pour déplacer la colonne (22) dans le plan de réglage.

2. Dispositif (10) selon la revendication 1,
**caractérisé en ce que** la partie de crochet (36) du support de fémur (18) présente une portion (40) droite, dépassant perpendiculairement de la tige (38), et une portion (42) substantiellement en forme de S, adjacente à la portion droite (40), avec une extrémité (44) constituant la base pour le fémur à fixer.

3. Dispositif (10) selon la revendication 2,
**caractérisé en ce que** l'extrémité (44) de la portion (42) en forme de S est arrondie.

4. Dispositif (10) selon la revendication 2 ou 3,
**caractérisé en ce que** la tige (38), la portion droite (40) de la partie de crochet (36) et une partie de la portion (42) en forme de S, adjacente à la portion droite (40), sont situées dans un plan (A-A), et
l'extrémité (44) de la portion (42) en forme de S est courbée dans une direction s'éloignant dudit plan (A-A).

5. Dispositif (10) selon l'une quelconque des revendications précédentes, **caractérisé en ce que** la partie de crochet (36) du support de fémur (18) est formée à partir d'un profilé présentant une section transversale de profilé plane.

6. Dispositif (10) selon la revendication 5,
**caractérisé en ce que** la section transversale de profilé plane se situe dans la zone de l'extrémité (44) à l'opposé de la section transversale de profilé plane dans la zone de la partie de crochet (36) restante, pivotée d'un angle entre 10° et 30°.

7. Dispositif (10) selon l'une quelconque des revendications précédentes, **caractérisé en ce que** la bride (30) présente au moins une douille (94, 96) pour recevoir le logement de la colonne (22) du bras de levier (20) par complémentarité de forme.

8. Dispositif (10) selon la revendication 7,
**caractérisé en ce que** la douille (94, 96) et la colonne (22) présentent des sections transversales polygonales correspondant les unes aux autres et présentant chacune une symétrie telle que la colonne (22) peut être reçue dans au moins deux orientations différentes dans la douille (94, 96), et
la colonne (22) présente deux portions de colonne (100, 102) droites, décalées en parallèle l'une par rapport à l'autre et reliées ensemble par un adaptateur coudé (104).

9. Dispositif (10) selon l'une quelconque des revendications précédentes, **caractérisé en ce que** la bride (30) présente un rail profilé (88) monté sur la table d'opération, et **en ce que** la bride (30) présente au moins une saillie en forme de griffe (74, 76), une pièce de serrage (78) précontrainte dans une position ouverte par un élément de ressort (82) et un élément de commande (84) permettant de déplacer la pièce de serrage (78) dans une position fermée en diminuant sa distance par rapport à la saillie (74, 76) contre la force de précontrainte exercée par l'élément de ressort (82), le rail profilé (88) dans la position ouverte pouvant être reçu dans l'espace entre la saillie (74, 76) et la pièce de serrage (78) et étant bloqué entre la saillie (74, 76) et la pièce de serrage (78) dans la position fermée.

10. Dispositif (10) selon la revendication 9,
**caractérisé en ce que** la pièce de serrage (78) est montée pivotante dans un axe de palier (80) entre sa position ouverte et sa position fermée, et **en ce que** l'élément de commande est une poignée vissée (84) à commande manuelle qui pour faire pivoter la pièce de serrage (80) dans la position fermée exerce une pression sur la pièce de serrage (78) contre la force de précontrainte exercée par l'élément de ressort (82).

11. Dispositif (10) selon l'une quelconque des revendications précédentes, **caractérisé en ce que** la console (24) est accouplée à la colonne (22) de manière à pouvoir pivoter autour de l'axe longitudinal (D) de celle-ci.

12. Dispositif (10) selon la revendication 11,
**caractérisé en ce que** la console (24) présente une première surface de contact (66) et la colonne (22) présente une deuxième surface de contact (68) tournée vers la première surface de contact, et **en ce que** les deux surfaces de contact (66, 68) peuvent être mises en appui l'une contre l'autre pour bloquer l'orientabilité de la console (24).

13. Dispositif (10) selon l'une quelconque des revendications précédentes, **caractérisé en ce que** la tige (38) est une portion d'extrémité bombée (58) et le logement de la console (24) est formée par une rainure (60) longitudinale dans laquelle la portion d'extrémité bombée (58) de la tige (38) peut s'emboîter.

14. Dispositif (10) selon l'une quelconque des revendications précédentes, **caractérisé en ce que** la console (24') se compose d'au moins deux parties (108, 110) dont une partie (11) est mobile contre l'autre partie (108) de manière télescopique pour régler la longueur de la console (24').

15. Dispositif (10) selon la revendication 14,
**caractérisé en ce que** l'une des deux parties (108, 110) de la console (24) présente un élément d'arrêt et l'autre partie présente plusieurs orifices d'arrêt (112) pour recevoir l'élément d'arrêt (114).
